# EUROPÄISCHE PATENTSCHRIFT

(11) **EP 1 105 367 B1**
(45) Veröffentlichungstag und Bekanntmachung des Hinweises auf die Patenterteilung: **18.06.2003**
(21) Anmeldenummer: 99940172.2
(22) Anmeldetag: 09.08.1999
(51) Int. Cl.: C07C 69/76, C07C 63/68, C07C 67/343, C07C 51/353, C07C 67/303, C07C 51/36, C07C 67/313, C07C 51/373, C07C 62/38, C07C 69/757

(54) **SUBSTITUIERTE ZIMTSÄUREN UND ZIMTSÄUREESTER**
SUBSTITUTED CINNAMIC ACIDS AND CINNAMIC ACID ESTERS
ACIDES CINNAMIQUES ET ESTERS D'ACIDE CINNAMIQUE SUBSTITU S

(30) Priorität: 17.08.1998 DE 19837069
(43) Veröffentlichungstag der Anmeldung: 13.06.2001
(73) Patentinhaber: Bayer Aktiengesellschaft, 51368 Leverkusen (DE)
(72) Erfinder: LANGE, Walter, D-50733 Köln (DE); KOMOSCHINSKI, Joachim, D-51061 Köln (DE); STEFFAN, Guido, D-51519 Odenthal (DE); KYSELA, Ernst, D-51427 Bergisch Gladbach (DE)
(86) Internationale Anmeldenummer: EP9905735
(87) Internationale Veröffentlichungsnummer: WO00010963

(56) Entgegenhaltungen:
- EP-A- 0 606 057
- US-A- 5 300 675

## Beschreibung

Die vorliegende Erfindung betrifft substituierte Zimtsäuren und Zimtsäureester, ein Verfahren zu ihrer Herstellung und ihre Verwendung zur Synthese von Insektizidvorprodukten.

Der Synthese von Insektiziden kommt hohe Bedeutung zu. Wichtige Zwischenprodukte bei dieser Synthese sind die substituierten Indanoncarbonsäureester sowie deren Salze.

Aus der WO 95/29171 ist beispielsweise ein Verfahren bekannt zur Herstellung von Oxadiazinen, die im Bereich des Pflanzenschutzes zur Bekämpfung von Arthropoden eingesetzt werden. Im Rahmen des mehrstufigen Herstellungsprozesses kommen auch substituierte Indanoncarbonsäureester als Zwischenprodukte zum Einsatz. Die Synthese der substituierten Indanoncarbonsäureester umfaßt eine Friedel-Crafts Reaktion von para-substituierten Phenylacetylhalogeniden mit Ethylen in Gegenwart einer Lewis-Säure und einem inerten Lösungsmittel unter Bildung eines 2-Tetralons der Formel A, wobei R' für F, Cl oder C₁-C₃-Fluoralkoxy steht, 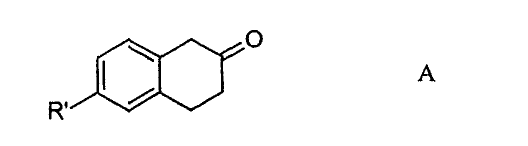 die Umsetzung der Verbindung A mit Peroxycarbonsäuren unter Bildung von substituierten Arylpropionsäuren der Formel B, 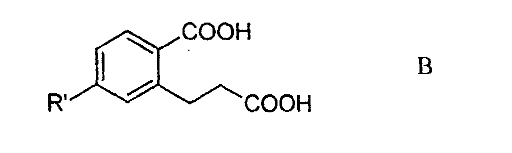 die Veresterung der substituierten Arylpropionsäuren der Formel B mit C₁-C₃-Alkoholen in Gegenwart eines Säurekatalysators unter Bildung der substituierten Arylpropionsäureester der Formel C, wobei R" für C₁-C₃-Alkyl steht, 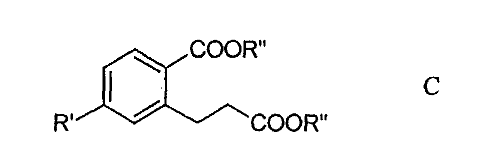 und die Reaktion der Verbindungen C mit einer Base unter Ringschluß und Bildung des substituierten Indanoncarbonsäureesters der Formel D

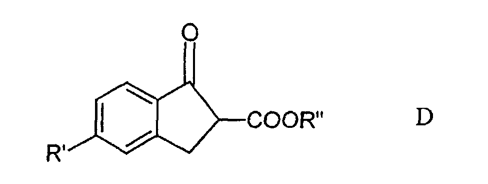

Nachteilig an dieser Synthese der substituierten Indanoncarbonsäureester ist, daß für die Umsetzung der Phenylacetylhalogenide mit Ethylen in der Friedel-Crafts-Reaktion die Zugabe von 0,9 bis 1,5 Mol-Äquivalenten einer Lewis-Säure, wie z.B. Aluminiumtrichlorid, notwendig ist. Hierdurch fallen bei der Aufarbeitung des Reaktionsgemisches große Mengen an Salzen und damit entsprechende Volumina belasteter Abwässer an. Hinzukommt, daß diese Synthese die Verwendung von Peroxycarbonsäuren, wie z.B. Peroxyessigsäure, zur Spaltung des 2-Tetralons erfordert. Die Peroxycarbonsäuren müssen dabei in einer Menge von 2,5 bis 3,5 Mol-Äquivalenten eingesetzt werden. Dies zieht bei einer Durchführung der Reaktion in technischem Maßstab Sicherheitsrisiken nach sich. So ist auf die exakte Einhaltung der Reaktionstemperatur zu achten und ferner die Zugabe der Peroxycarbonsäure genauestens zu kontrollieren, um eine Akkumulation zu hoher Mengen überschüssiger Peroxycarbonsäure im Reaktionssystem zu vermeiden.

Aus J. Pharm. Sci. 67(1) 1978, 81 ist es ferner bekannt, den Chlor-substituierten Indanoncarbonsäureester 5-Chlor-2-methoxycarbonyl-1-indanon, ausgehend von 3-Chlorbenzaldehyd herzustellen. Hierbei wird zunächst 3-Chlorbenzaldehyd in Pyridin mit Malonsäure zur 3-Chlorzimtsäure umgesetzt. Nach Hydrierung der ethylenischen Doppelbindung sowie Cyclisierung zum 5-Chlor-1-indanon wird dieses mit Dimethylcarbonat in Gegenwart von Natriumhydrid sowie Benzol als Lösungsmittel zum 5-Chlor-2-methoxycarbonyl-1-indanon umgesetzt. Nachteilig an dieser Synthesemethode ist der vielstufige Mechanismus, durch den die Wahrscheinlichkeit zur Bildung verschiedener Nebenprodukte deutlich erhöht wird, was sich in einer nur geringen Ausbeute von 48% widerspiegelt. Hinzu kommt, daß die Gesamtreaktion mehrfach den Einsatz von teuren, sicherheitstechnisch problematischen oder gesundheitsgefährdenden Substanzen wie Natriumhydrid und Benzol erfordert.

Aus Chemical Abstracts 97, 1982, 109843f ist 5-Brom-2-carboxy-zimtsäure bekannt, die durch oxidative Spaltung von 6-Brom-2-naphthol erhalten und nachfolgend cyclisiert wird, mit PCl₅ und NH₃ zum Amid reagiert und abschließend unter Ringvergrößerung in Gegenwart von NaOCI zum 6-Brom-isochinolin-1-on umgesetzt wird.

Auch aus Chemical Abstracts 82, 1975, 31200n und 79, 1973, 91891m ist die oxidative Spaltung von 6-Brom-2-naphthol unter Bildung von 5-Brom-2-carboxyzimtsäure bekannt, die über mehrere Schritte der Amidierung, Hoffmann-Umlagerung und Cyclisierung zu den entsprechenden sustituierten Indolen führt.

Aus WO 97/43287 Al sind allgemein substituierte Zimtsäuren und Zimtsäurechloride bekannt, die am Phenylring durch einen Rest R¹ und ein oder zwei weitere Reste R² substituiert sein können, wobei für diese Reste eine Vielzahl von Bedeutungen möglich sind. Beschrieben wird ferner die Umsetzung der substituierten Zimtsäuren und Zimtsäurechloride mit anderen komplexen Edukten zu speziellen Carbolin-Derivaten umgesetzt, die als cGMP-PDE-Inhibitoren für Herzkreislauf-Indikationen Anwendung finden.

Aus WO 96/04241 Al ist in Form der Preparation 45 eine Zimtsäure bekant, die in einer m-Stellung des 2-Carboxyvinyl-Rests durch Methylcarboxylat und in der anderen m-Stellung durch Iod substituiert ist. Das Augenmerk der WO 96/04241 ist auf die Herstellung von speziellen, pharmazeutisch wirksamen Benzoylguanidin-Derivaten gerichtet, in der auch die Preparation 45 verwendet wird.

Aus EP-A-0 508 264 ist ein Verfahren zur Herstellung von Arylolefinen mit einer breiten Definition bekannt, die allgemein auch substituierte Zimtsäuren und Zimtsäureester umfaßt. Die herstellbaren Arylolefine finden in ganz unterschiedlichen Bereichen Anwendung, z.B. als optische Aufheller, als Vorprodukte für optische Aufheller, als Zwischenprodukte für Pharmazeutika oder als UV-Absorber.

Die Aufgabe der vorliegenden Erfindung bestand daher darin, Zwischenprodukte bereitzustellen, aus denen in verfahrenstechnisch einfacher und sicherheitstechnisch ungefährlicher Weise die Synthese von substituierten Indanoncarbonsäureestern möglich wird.

Gelöst wird diese Aufgabe durch substituierte Zimtsäuren und Zimtsäureester der Formel (I) 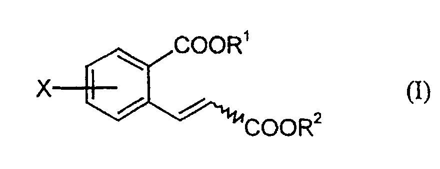 wobei X für F, Cl oder J steht und R¹ und R² gleich oder verschieden sind und für Wasserstoff, einen C₁-C₁₀-Alkylrest oder einen Benzylrest stehen.

Diese substituierten Zimtsäuren bzw. Zimtsäureester zeichnen sich dadurch aus, daß sie erstmals in einem unerwartet einfachen Zweistufenverfahren eine kostengünstige Synthese von substituierten Indanoncarbonsäureestern ermöglichen.

In den substituierten Zimtsäuren bzw. Zimtsäureestern steht X für F, Cl oder J, bevorzugt für Chlor.

R¹ und R² sind gleich oder verschieden und stehen für Wasserstoff, einen gegebenenfalls substituierten C₁-C₁₀-Alkylrest oder einen gegebenenfalls substituierten Benzylrest. Bevorzugt bedeuten R¹ und R² unabhängig voneinander Wasserstoff, Methyl, Ethyl, Propyl, i-Propyl, n-Butyl, i- Butyl, tert. Butyl, n-Pentyl, i-Pentyl, n-Hexyl, i-Hexyl, n-Heptyl, i-Heptyl, n-Octyl, i- Octyl, n-Nonyl, i-Nonyl, n-Decyl oder i-Decyl. Insbesondere stehen R¹ und R² unabhängig voneinander für Wasserstoff oder Methyl.

Für den Fall, daß der C₁-C₁₀-Alkylrest als Rest R¹ oder R² substituiert ist, kann es sich bei diesen Substituenten um Halogen, Hydroxy oder C₆-C₁₂-Arylreste handeln. Der Benzylrest als Rest R¹ oder R² kann durch Halogen-, Hydroxy-, C₁-C₁₀-Alkyloder C₆-C₁₂-Arylreste substituiert sein.

Der Substituent X befindet sich in den Zimtsäuren bzw. Zimtsäureestern der Formel (I) bevorzugt in 5-Stellung zum Acrylsäure- bzw. Acrylsäureesterrest.

Bevorzugte Verbindungen der Formel (I) sind 4-Chlor-2-(3-methoxy-3-oxo-1-propenyl)-benzoesäuremethylester, 4-Chlor-2-(3-methoxy-3-oxo-1-propenyl)-benzoesäure, 4-Fluor-2-(3-methoxy-3-oxo-1-propenyl)-benzoesäure, 4-Chlor-2-(3-hydroxy-3-oxo-1-propenyl)-benzoesäuremethylester sowie 4-Chlor-2-(3-hydroxy-3-oxo-1-propenyl)-benzoesäure.

Die Herstellung der substituierten Zimtsäuren bzw. Zimtsäureester der Formel (I) ist über eine Abwandlung der Heck-Reaktion möglich (Verfahren A).

Gegenstand der Erfindung ist ein Verfahren zur Herstellung der substituierten Zimtsäuren und Zimtsäureester der Formel (I) durch Umsetzung von Diazonium-Salzen der Formel (II) mit Acrylsäure-Derivaten der Formel (III) in Gegenwart eines Palladium-haltigen Katalysators, wobei X, R¹ und R² die in der Formel (I) genannten Bedeutungen haben und A- für Halogenid, bevorzugt Chlorid oder Bromid, Sulfat, Hydrogensulfat, Nitrat, Phosphat, Acetat oder Tetrafluoroborat steht, dadurch gekennzeichnet, daß die Umsetzung in Abwesenheit von Basen durchgeführt wird. Dieser Syntheseweg ist besonders vorteilhaft und daher bevorzugt.

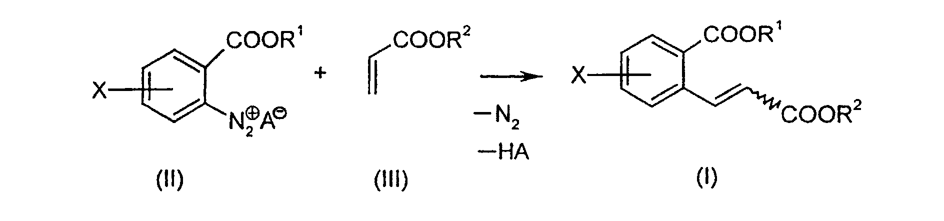

X, R¹ und R² haben bevorzugt die auch für die Formel (I) als bevorzugt genannten Bedeutungen. A⁻ steht bevorzugt für Chlorid, Sulfat, Hydrogensulfat, Acetat oder Tetrafluoroborat.

Das Reaktionsprinzip dieses Verfahrens A ist generell als Matsuda Variante der Heck-Reaktion bekannt. Gemäß EP-A-0 584 043 können z.B. ganz allgemein Verbindungen der Formel Ar-CHRₐ-CHR_{b}R_{c} hergestellt werden, wobei Rₐ, R_{b} und R_{c} unabhängig voneinander Wasserstoff oder einen hydrier-inerten Substituenten bedeuten und Ar einen gegebenenfalls substituierten C₆-C₂₀-Aryl- oder C₃-C₂₀-Heteroarylrest darstellt. Hierzu wird in einem ersten Schritt 1 Mol-Äquivalent des Diazonium-Kations Ar-N₂⁺ mit mindestens 1 Mol-Äquivalent einer Verbindung CRₐ=CR_{b}R_{c} unter Bildung der Verbindung Ar-CHRₐ=CHR_{b}R_{c} umgesetzt, wobei in Gegenwart einer katalytischen Menge eines homogenen Palladium-Katalysators gearbeitet wird. Ferner ist der Zusatz einer Base zwingend erforderlich. Die Zugabe von 1 bis 10 Mol-Äquivalenten dieser Base zieht insbesondere bei der technischen Durchführung der Heck-Reaktion zusätzliche Kosten und eine aufwendige Aufarbeitung des Reaktionsgemisches nach sich. Die Verbindung Ar-CHRₐ=CHR_{b}R_{c} wird in einem zweiten Schritt zur Verbindung Ar-CHRₐ-CHR_{b}R_{c} hydriert. Dieser Hydrierschritt zeichnet sich dadurch aus, daß in Gegenwart katalytischer Mengen eines heterogenen Palladium-Katalysators gearbeitet wird, der aus dem homogenen Palladium-Katalysator des ersten Schritts durch Reduktion vor dem zweiten Schritt erhalten wird.

EP-A-0 584 043 offenbart explizit und schwerpunktmäßig nur solche Verbindungen Ar-HRₐ=CHR_{b}R_{c} und Ar-CHRₐ-CHR_{b}R_{c}, die am Aryl-Rest Ar eine Sulfonsäuregruppe und gegebenenfalls weitere Substituenten tragen. EP-A-0 584 043 offenbart jedoch weder die erfindungsgemäßen Zimtsäuren bzw. Zimtsäureester der Formel (I), die am Rest Ar = Phenyl durch einen Halogenrest sowie einen Carbonsäure- bzw. Carbonsäureesterrest substituiert sind, noch deren spezielle Herstellung gemäß Verfahren A noch deren ausgezeichnete Eignung als Edukte für die Herstellung von substituierten Indanoncarbonsäureestern. Das Verfahren A zeichnet sich im Vergleich zum Verfahren der EP-A-0 584 043 dadurch aus, daß die Erzielung exzellent hoher Ausbeuten auch ohne den Zusatz einer Base möglich ist, wodurch die wirtschaftliche Attraktivität des Verfahrens im Hinblick auf Aufarbeitung und Abwasserentstehung gesteigert wird. Es kann sogar in mineral- oder schwefelsaurer Lösung gearbeitet werden.

Auch aus der EP-A-0 508 264 ist das Prinzip des Verfahrens A bereits bekannt, aus Aryldiazoniumsalzen und Olefinen in Gegenwart eines Palladium-Katalysators die entsprechenden Arylolefine herzustellen. Der Schwerpunkt der EP-A-0 508 264 liegt jedoch wie im Fall der EP-A-0 584 043 auf Verbindungen, die am Arylrest eine Sulfonsäuregruppe tragen. Eine explizite Offenbarung der ausgewählten erfindungsgemäßen Zimtsäuren bzw. Zimtsäureester und deren Eignung zur Herstellung von substituierten Indanoncarbonsäureestern ist nicht vorhanden. Gemäß EP-A-0 508 264 werden in der Heck-Reaktion ebenfalls Basen zugesetzt sowie günstigerweise Liganden, wie Triarylphosphane oder Bis(diarylphosphan)alkane, die mit dem Palladium oder den Palladiumsalzen Komplexe bilden können. Das Verfahren A zeichnet sich demgegenüber aus, daß die Zugabe solcher Hilfsliganden zum Katalysator üblicherweise nicht erforderlich ist.

Die Heck Reaktion nach Variante A wird durchgeführt unter Verwendung von Palladium(II)salzen, wie PdCl₂, PdBr₂, Pd(NO₃)₂, H₂PdCl₄, Pd(CH₃COO)₂, [PdCl₄]Na₂, [PdCl₄]Li₂, [PdCl₄]K₂, oder Palladium(II)acetylacetonat. PdCl₂, Pd(CH₃COO)₂ und Palladium(II)acetylacetonat sind besonders bevorzugt. Üblicherweise werden 0,001-10 mol% des Palladium-haltigen Katalysators, bezogen auf das Diazonium-Salz der Formel (II), eingesetzt.

Die Reaktionstemperatur für die Variante A sollte unterhalb der Zersetzungstemperatur des Diazoniumions liegen, in der Regel wird bei -20°C bis 100°C, bevorzugt 20 bis 80°C und insbesondere 40 bis 65°C gearbeitet. Die Reaktion kann unter Zusatz geeigneter Lösungsmittel erfolgen, üblicherweise werden Wasser, Alkohole, bevorzugt Methanol, Ethanol, Propanol, i-Propanol oder Butanol, Ameisensäure, Tetrahydrofuran oder Acetonitril eingesetzt.

Die im Verfahren A eingesetzten Diazoniumsalze der Formel (II) sind durch Umsetzung von halogenierten Anthranilsäure-Derivaten mit Natriumnitrit in saurer wässriger Lösung oder mit Methylnitrit in saurem Methanol zugänglich. Bei Verwendung von Natriumnitrit in bevorzugt schwefelsaurer wässriger Lösung können zusätzlich auch noch geringe Mengen Isopropanol zugegen sein. Beim Einsatz von Methylnitrit in schwefelsaurem Methanol wird in der Regel wasserfrei gearbeitet, um eine unnötige Hydrolyse von Methylnitrit zu vermeiden. Es ist von Vorteil, daß bei dieser Diazotierung keine organischen Lösungsmittel wie Dimethylformamid (DMF), N-Methylpyrrolidon (NMP) oder Dimethylsulfoxid (DMSO) verwendet werden müssen. Von Vorteil sind ferner die niedrigen Reaktionstemperaturen. Üblicherweise fallen die Zimtsäuren bzw. Zimtsäureester der Formel (I) aus einer wässrigen Reaktionsmischung aus oder können durch zusätzliche Wasserzugabe ausgefällt werden. Die erhaltenen Feststoffe können für Folgereaktionen durch Zugabe von organischen Lösungsmitteln in Lösung gebracht werden.

Anstelle der Diazonium-Salze der Formel (II) können in der Heck-Kupplung auch Halogenaromaten der Formel (IV) mit den Acrylsäure-Derivaten der Formel (III) umgesetzt werden (Verfahren B), wobei X, R¹ und R² die für die Formel (I) angegebenen Bedeutungen haben. Y steht für Brom oder Jod. Auch aus EP-A-0 688 757 ist die Umsetzung von solchen Halogenaromaten mit Olefinen bekannt, wobei als Palladium-Katalysatoren spezielle Palladacyclen, insbesondere µ-verbrückte DiPalladium-Komplexe, eingesetzt werden.

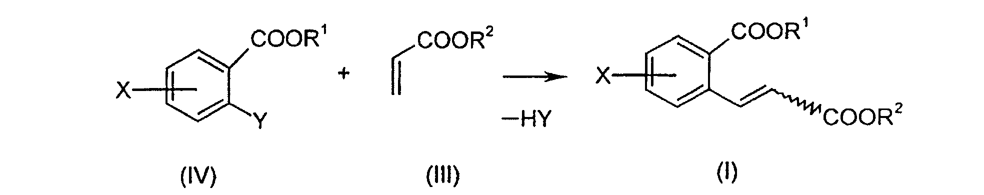

Gegenstand der vorliegenden Erfindung ist ferner die Verwendung der substituierten Zimtsäuren und Zimtsäureester der Formel (I) zur Herstellung von substituierten Indanoncarbonsäureestern der Formel (VII) 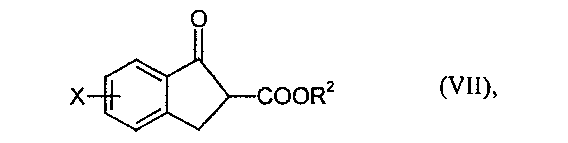 wobei
X und R² die für die Formel (I) genannten Bedeutungen besitzen.

Eine bevorzugte Ausführungsform dieser Verwendung ist dadurch gekennzeichnet, daß die substituierten Zimtsäuren und Zimtsäureester der Formel (I) in einem ersten Schritt in Gegenwart eines Hydrierkatalysators mit Wasserstoff hydriert werden unter Bildung von substituierten Arylpropionsäuren der Formel (VIII), 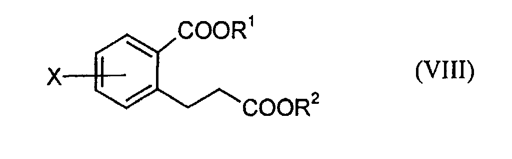 welche dann in einem zweiten Schritt in Gegenwart einer Base unter Bildung der substituierten Indanoncarbonsäureester der Formel (VII) cyclisiert werden, wobei X, R¹ und R² in den Formeln (VII) und (VIII) jeweils die für die Formel (I) genannten Bedeutungen besitzen.

Die gemäß den Verfahren A oder B erhaltenen erfindungsgemäßen Zimtsäuren bzw. Zimtsäureester der Formel (I) können mit oder ohne vorhergehende Isolierung aus den jeweiligen Reaktionsgemischen in die erste Stufe zur Synthese der substituierten Indanoncarbonsäureester eingebracht werden. Werden die Zimtsäuren bzw. Zimtsäureester der Formel (I) nach ihrer Herstellung über das Verfahren A oder B nicht isoliert, sondern das gesamte Reaktionsgemisch zur Herstellung des Indanoncarbonsäureesters der Formel (VII) eingesetzt, so fungiert der Palladium-Katalysator der Heck-Reaktion als Hydrierkatalysator für die Herstellung der Arylpropionsäuren der Formel (VIII). Werden die Zimtsäuren bzw. Zimtsäureester aus dem Reaktionsgemisch des Verfahrens A oder B als Feststoffe isoliert, so kann für die Hydrierung zu den Arylpropionsäuren ein Hydrierkatalysator zugegeben werden. Die ist jedoch auch nicht in allen Fällen erforderlich, wenn der isolierte Feststoff noch geringe Mengen des in der Heck-Reaktion verwendeten Katalysators enthält. Wird ein Hydrierkatalysator noch zusätzlich zugegeben, so handelt es sich üblicherweise um einen auf Aktivkohle aufgebrachten Palladium- oder Platin-Katalysator.

Die Hydrierung zu den gesättigten Arylpropionsäuren der Formel (VIII) erfolgt in Gegenwart von Wasserstoff sowie üblicherweise Wasser, Mineralsäuren und/oder Alkoholen als Lösungsmittel.

Als Mineralsäure ist üblicherweise Schwefelsäure zugegen, wenn die Zimtsäuren bzw. Zimtsäureester vor ihrer Hydrierung nicht aus dem Reaktionsgemisch der vorhergehenden Verfahren isoliert werden. Als Alkohole können beispielsweise Methanol, Ethanol, Propanol, i-Propanol oder Xylol anwesend sein.

Üblicherweise wird die Hydrierung unter einem Druck von 1-100 bar durchgeführt.

Die Cyclisierung der substituierten Arylpropionsäuren der Formel (VIII) zu den entsprechend substituierten Indanoncarbonsäureestern der Formel (VII) wird in Gegenwart einer starken Base und eines geeigneten Lösungsmittels durchgerührt. Üblicherweise wird als starke Base ein Alkalimetallhydrid, bevorzugt Natriumhydrid oder ein Alkalimetallalkoholat, bevorzugt Natriumalkoholat, eingesetzt. Als Lösungsmittel haben sich Toluol, Xylol, Benzol oder die den Alkalimetallalkoholaten entsprechenden Alkohole bewährt. Insbesondere wird Xylol oder Methanol eingesetzt. Bei einer Reaktionstemperatur von 60 bis 90°C, einem Reaktionsdruck von 100 bis 500 kPa beträgt die Reaktionszeit 0,5 bis 10 Stunden. Die Indanoncarbonsäureester fallen hierbei als Alkalimetallsalz an und werden zunächst durch Zugabe einer Säure wie z.B. Eisessig oder verdünnte wässrige Mineralsäure neutralisiert und anschließend durch Filtration oder Extraktion isoliert. Im übrigen wird für die Reaktionsbedingungen der Cyclisierung der substituierten Arylpropionsäuren der Formel (VIII) auf die entsprechende Offenbarung der WO 95/29 171 verwiesen, auf die hiermit ausdrücklich Bezug genommen wird.

### Beispiel 1

### 4-Chlor-2-(3-methoxy-3-oxo-1-propenyl)-benzoesäuremethylester

Aus 76 g Natriumnitrit wird in 55 ml Methanol und 183 ml Wasser mit 80 ml 50 %iger Schwefelsäure Methylnitrit erzeugt, das bei 10 bis 15°C in eine Mischung aus 171,5g 2-Amino-4-chlorbenzoesäure, 800ml Methanol und 200ml konzentrierte Schwefelsäure eingeleitet wird. Nach einstündigem Rühren werden 8 g Amidosulfonsäure zugesetzt und überschüssiges Methylnitrit durch Überleiten eines Stickstoffstroms aus dem Reaktionsgemisch entfernt. Anschließend gibt man 86 g Methylacrylat sowie 666 mg Palladiumacetylacetonat zu und erhitzt 3 Stunden auf 40°C. Anschließend setzt man 1 600 ml Methanol zu und erhitzt weitere 3 Stunden zum Sieden. Nach Zugabe von 2 500 ml Eiswasser wird der ausgefallene Niederschlag abfiltriert, mit Wasser gewaschen und anschließend getrocknet. Es werden 207 g 4-Chlor-2-(3-methoxy-3-oxo-1-propenyl)-benzoesäuremethylester mit einem Schmelzpunkt von 87 bis 89°C erhalten.

### Beispiel 2

### 4-Fluor-2-(3-methoxy-3 -oxo-1-propenyl)-benzoesäure

Bei 0°C wird innerhalb von 30 Minuten eine Lösung von 0,52 g Natriumnitrit in 1 ml Wasser zu einer Mischung aus 1 g 2-Amino-4-fluorbenzoesäure, 15 ml Wasser und 6,24 ml konzentrierter Schwefelsäure zugegeben. Anschließend wird eine Stunde gerührt. Nach Zugabe von 0,3 g Amidosulfonsäure tropft man 0,7 g Methylacrylat in 9 ml Isopropanol zu und gibt 10 mg Palladiumacetylacetonat zu. Nach 4 Stunden bei 40°C gibt man 30 ml Wasser zu und filtriert den ausgefallenen Niederschlag ab. Nach dem Trocknen erhält man 1,1g 4-Fluor-2-(3-methoxy-3-oxo-1-propenyl)-benzoesäure mit einem Schmelzpunkt von 151 bis 153°C.

### Beispiel 3

### 4-Chlor-2-(3-methoxy-3-oxo-1-propenyl)-benzoesäuremethylester

Bei 2°C wird eine Lösung von 79,5 g Natriumnitrit in 150 ml Wasser zu einer Mischung aus 185,5 g 2-Amino-4-chlorbenzoesäuremethylester, 785 ml Wasser und 190,5 ml konzentrierte Schwefelsäure zugegeben. Anschließend rührt man 30 Minuten und gibt dann 5,3 g Amidosulfonsäure zu. Das erhaltene Reaktionsgemisch wird zu 108,2 g Methylacrylat zugetropft. Anschließend gibt man 766 mg Palladiumacetylacetonat zur Reaktionsmischung und erhitzt auf 40°C. Nach weiteren 3,5 Stunden bei 40°C filtriert man den ausgefallenen Niederschlag ab und erhält nach Trocknung 248,3 g 4-Chlor-2-(3-methoxy-3-oxo-1-propenyl)-benzoesäuremethylester mit einem Schmelzpunkt von 87 bis 89°C.

### Beispiel 4

### 4-Chlor-2-(3-hydroxy-3-oxo-1-propenyl)-benzoesäuremethylester

Bei 2°C wird eine Lösung von 5,96 g Natriumnitrit in 11,3 ml Wasser zu einer Mischung aus 14 g 2-Amino-4-chlorbenzoesäuremethylester, 59 ml Wasser und 14,3 ml konzentrierter Schwefelsäure gegeben. Anschließend rührt man 20 Minuten und gibt die Reaktionsmischung dann portionsweise zu 6,9 g Acrylsäure, wobei man nach der ersten Portion 57 mg Palladiumacetylacetonat zusetzt. Nach 4 Stunden bei 40°C filtriert man den ausgefallenen Feststoff ab und erhält nach Waschen und Trocknen 18,5 g 4-Chlor-2-(3-hydroxy-3-oxo-1-propenyl)-benzoesäuremethylester.

### Beispiel 5

### 4-Chlor-2-(3-hydroxy-3-oxo-1-propenyl)-benzoesäure

Bei 2°C wird eine Lösung von 2,98 g Natriumnitrit in 5,65 ml Wasser zu einer Mischung aus 6,43 g 2-Amino-4-chlor-benzoesäure, 29,5 ml Wasser und 14,3 ml konzentrierter Schwefelsäure gegeben. Anschließend gibt man 0,4 g Amidosulfonsäure zu und tropft diese Reaktionsmischung dann zu 2,7 g Acrylsäure und 28,5 mg Palladiumacetylacetonat. Nach 4 Stunden bei 40°C filtriert man den ausgefallenen Feststoff ab und erhält 7,48 g 4-Chlor-2-(3-hydroxy-3-oxo-1-propenyl)-benzoesäure mit einem Schmelzpunkt von 200 bis 202°C.

Bei analoger Durchführung wird bei Verwendung von 21 mg Palladiumacetat anstelle von 28,5 mg Palladiumacetylacetonat 6,7 g Produkt erhalten.

### Beispiel 6

### 4-Chlor-2-(3-methoxy-3-oxo-1-propanyl)-benzoesäuremethylester

5 g 4-Chlor-2-(3-hydroxy-3-oxo-1-propenyl)-benzoesäuremethylester in Form des isolierten Feststoffs aus Beispiel 4 werden in 75 ml Methanol bei 20 bis 30°C und 20 bar Wasserstoffdruck hydriert, wobei kein Hydrierkatalysator zugesetzt wird. Nach Filtration über Celite® und Entfernung der Lösungsmittel unter Vakuum erhält man 3,3 g Produkt, daß nach Gaschromatographie 88 % des 4-Chlor-2-(3-methoxy-3-oxo-1-propenyl)-benzoesäuremethylester enthält.

Das gleiche Produkt wird erhalten, wenn man statt 4-Chlor-2-(3-hydroxy-3-oxo-1-propenyl)-benzoesäuremethylester 4-Chlor-2-(3-methoxy-3-oxo-1-propenyl)-benzoesäuremethylester aus Beispiel 1 einsetzt.

### Beispiel 7

### 4-Chlor-2-(3-methoxy-3-oxo-1-propanyl)-benzoesäure

43 g 4-Chlor-2-(3-hydroxy-3-oxo-1-propenyl)-benzoesäure in Form des isolierten Feststoffs aus Beispiel 5 werden in 400 ml Methanol bei 40°C und 20 bis 40 bar Wasserstoffdruck hydriert, ohne daß ein zusätzlicher Hydrierkatalysator zugesetzt wird. Nach Filtration über Celite® und Entfernung der Lösungsmittel unter Vakuum werden 35,3 g Produkt erhalten, daß nach Gaschromatographie 81 % des 4-Chlor-2-(3-methoxy-3-oxo-1-propenyl)-benzoesäure enthält.

Das gleiche Produkt wird erhalten, wenn man statt 4-Chlor-2-(3-hydroxy-3-oxo-1-propenyl)-benzoesäure 4-Chlor-2-(3-methoxy-3-oxo-1-propenyl)-benzoesäure einsetzt.

### Beispiel 8

### 4-Chlor-2-(3-methoxy-3-oxo-1-propanyl)-benzoesäuremethylester

26,1 g des nach Beispiel 1 hergestellten 4-Chlor-2-(3-methoxy-3-oxo-1-propenyl)-benzoesäuremethylesters werden erneut in 300 ml Methanol aufgenommen und in einen Autoklaven überführt und anschließend bei 30°C, einem Wasserstoffdruck von 30 bar 5 h bis zur theoretischen Wasserstoff-Aufnahme hydriert. Die Substanz enthält den für die Heck-Kupplung benötigten Palladium-Katalysators, so daß auf eine separate Zugabe eines Hydrierkatalysators verzichtet werden kann. Nach Reduktion des Drucks auf 1 bar filtriert man vom Ungelösten ab. Anschließend wird unter vermindertem Druck Lösungsmittel entfernt, bis das Hydrierprodukt ausfällt. Nach Umkristallisation erhält man 21 g 4-Chlor-2-(3-methoxy-3-oxo-1-propanyl)-benzoesäuremethylester.

### Beispiel 9

### 4-Chlor-2-(3-methoxy-3-oxo-1-propanyl)-benzoesäuremethylester

26,1 g des nach Beispiel 3 erhaltenen 4-Chlor-2-(3-methoxy-3-oxo-1-propenyl)-benzoesäuremethylesters werden in 250 ml Methanol aufgenommen, hierzu gibt man ca. 1 g Aktivkohle, rührt unter Rückfluß auf und filtriert heiß ab und entfernt mit diesem Schritt die Restmengen an Palladium Katalysator aus der Heck-Reaktion.

Zum wieder auf Raumtemperatur abgekühlten Filtrat gibt man nun 1,5 g eines 5 %igen Platin/Kohle-Katalysators und hydriert bei 30°C und 5 bar Wasserstoffdruck, ca. 4 bis 5 h bis zur theoretischen Wasserstoff-Aufnahme. Nach Reduktion des Drucks, Abfiltration des Katalysators und Entfernung des Lösungsmittels aus der Mischung erhält man auf diese Weise 25,2 g 4-Chlor-2-(3-methoxy-3-oxo-1-propanyl)-benzoesäureester.

### Beispiel 10

### 2-Carboxymethyl-5-chlorindan-1-on

108g 4-Chlor-2-(3-methoxy-3-oxo-1-propenyl)-benzoesäuremethylester werden in 300 ml Methanol gelöst, anschließend gibt man 80 g Natriummethylat dazu. Es wird nun auf 70°C erhitzt und ein Teil des Methanols abdestilliert, so daß das Reaktionsgemisch noch rührfähig bleibt. Nach 2 h gibt man langsam 400 ml Toluol zu und entfernt das restliche Methanol. Anschließend wird noch 0,5 h nachgerührt, danach läßt man auf Raumtemperatur abkühlen. Zu der Mischung wird nun 10 g Essigsäure zugetropft, mit 500 ml Wasser verdünnt und mit 1 N Salzsäure auf pH 4 bis 5 eingestellt. Die Toluolphase wird bis zur Produktfällung eingeengt. Nach Filtration kann das Produkt aus Hexan umkristallisiert werden. Man erhält 93,5 g 2-Carboxymethyl-5-chlorindan-1-on.

## Patentansprüche

1. Substituierte Zimtsäuren und Zimtsäureester der Formel (I) 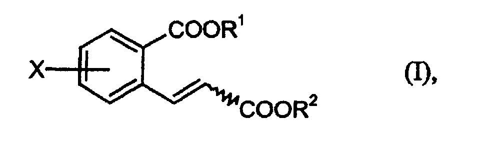 wobei X für F, Cl oder J steht und R¹ und R² gleich oder verschieden sind und für Wasserstoff, einen C₁-C₁₀-Alkylrest oder einen Benzylrest stehen.

2. Substituierte Zimtsäuren und Zimtsäureester nach Anspruch 1, **dadurch gekennzeichnet, daß** der C₁-C₁₀-Alkylrest als Rest R¹ oder R² durch Halogen, Hydroxy oder C₆-C₁₂-Arylreste substituiert ist und unabhängig davon der Benzylrest als Rest R¹ oder R² durch Halogen, Hydroxy, C₁-C₁₀-Alkyl- oder C₆-C₁₂-Arylreste substituiert ist.

3. Substituierte Zimtsäuren und Zimtsäureester nach Anspruch 2, **dadurch gekennzeichnet, daß** X für Chlor steht und unabhängig davon R¹ und R² gleich oder verschieden sind und für Wasserstoff, Methyl, Ethyl, Propyl, i-Propyl, n-Butyl, i- Butyl, tert. Butyl, n-Pentyl, i-Pentyl, n-Hexyl, i-Hexyl, n-Heptyl, i-Heptyl, n-Octyl, i- Octyl, n-Nonyl, i-Nonyl, n-Decyl oder i-Decyl, bevorzugt für Wasserstoff oder Methyl stehen.

4. Substituierte Zimtsäuren und Zimtsäureester nach Anspruch 1, **dadurch gekennzeichnet, daß** es sich um 4-Chlor-2-(3-methoxy-3-oxo-1-propenyl)benzoesäuremethylester, 4-Chlor-2-(3-methoxy-3-oxo-1-propenyl)-benzoesäure, 4-Fluor-2-(3-methoxy-3-oxo-1-propenyl)benzoesäure, 4-Chlor-2-(3-hydroxy-3-oxo-1-propenyl)benzoesäuremethylester oder 4-Chlor-2-(3-hydroxy-3-oxo-1-propenyl)benzoesäure handelt.

5. Verfahren zur Herstellung der substituierten Zimtsäuren und Zimtsäureester der Formel (I) durch Umsetzung von Diazonium-Salzen der Formel (II) mit Acrylsäure-Derivaten der Formel (III) in Gegenwart eines Palladium-haltigen Katalysators, wobei X, R¹ und R² die in der Formel (I) genannten Bedeutungen haben und A^{⊖} für Halogenid, bevorzugt Chlorid oder Bromid, Sulfat, Hydrogensulfat, Nitrat, Phosphat, Acetat oder Tetrafluoroborat steht, **dadurch gekennzeichnet, daß** die Umsetzung in Abwesenheit von Basen durchgeführt wird

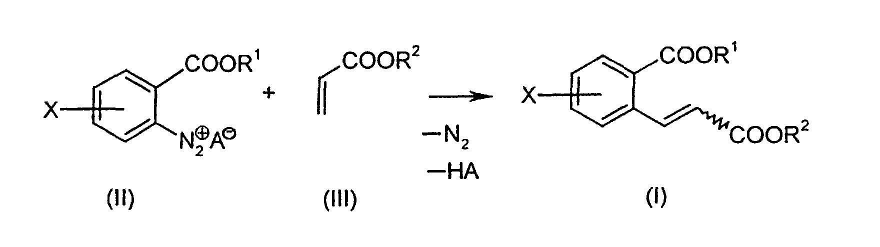

6. Verfahren nach Anspruch 5, **dadurch gekennzeichnet, daß** als Palladium-haltiger Katalysator Palladium(II)salze, bevorzugt PdCl₂, PdBr₂, Pd(NO₃)₂, H₂PdCl₄, Pd(CH₃COO)₂, [PdCl₄]Na₂, [PdCl₄]Li₂, [PdCl₄]K₂, oder Palladium(II)acetylacetonat eingesetzt werden.

7. Verfahren nach Anspruch 5 oder 6, **dadurch gekennzeichnet, daß** 0,001 bis 10 mol-% des Palladium-haltigen Katalysators bezogen auf das Diazonium-Salz der Formel (II) eingesetzt werden und unabhängig davon bei einer Temperatur von -20 bis 100°C, bevorzugt 20 bis 80°C und insbesondere 40 bis 65°C gearbeitet wird.

8. Verwendung der substituierten Zimtsäuren und Zimtsäureester nach einem oder mehreren der Ansprüche 1 bis 4 zur Herstellung von substituierten Indanoncarbonsäureestern der Formel (VII) 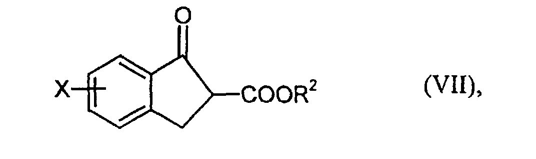 wobei X und R² in der Formel (VII) die für die Formel (I) genannten Bedeutungen besitzen.

9. Verwendung nach Anspruch 8, **dadurch gekennzeichnet, daß** die substituierten Zimtsäuren und Zimtsäureester der Formel (I) in einem ersten Schritt in Gegenwart eines Hydrierkatalysators mit Wasserstoff hydriert werden unter Bildung von substituierten Arylpropionsäuren der Formel (VIII), 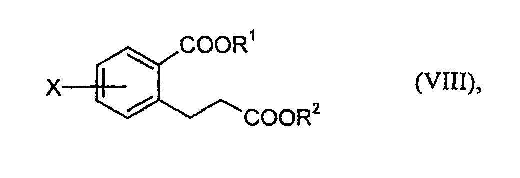 welche dann in einem zweiten Schritt in Gegenwart einer Base unter Bildung der substituierten Indanoncarbonsäureester der Formel (VII) cyclisiert werden, wobei X, R¹ und R² in den Formeln (VII) und (VIII) jeweils die für die Formel (I) genannten Bedeutungen besitzen.

10. Verwendung nach Anspruch 9, **dadurch gekennzeichnet, daß** die Hydrierung unter einem Druck von 1 bis 100 bar erfolgt und die Cyclisierung der substituierten Arylpropionsäuren der Formel (VIII) in Gegenwart eines Alkalimetallhydrids oder eines Alkalimetallalkoholats als Base durchgeführt wird.

## Claims

1. Substituted cinnamic acids and cinnamic acid esters of the formula (I) 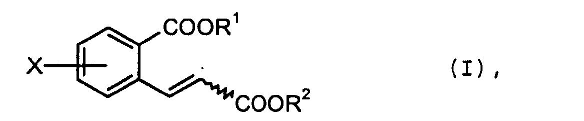 where X represents F, Cl or I and R¹ and R² are identical or different and represent hydrogen, a C₁-C₁₀-alkyl radical or a benzyl radical.

2. Substituted cinnamic acids and cinnamic acid esters according to Claim 1, **characterized in that** the C₁-C₁₀-alkyl radical as radical R¹ or R² is substituted by halogen, hydroxyl or C₆-C₁₂-aryl radicals and independently thereof the benzyl radical as radical R¹ or R² is substituted by halogen, hydroxyl, C₁-C₁₀-alkyl- or C₆-C₁₂-aryl radicals.

3. Substituted cinnamic acids and cinnamic acid esters according to Claim 2, **characterized in that** X represents chlorine and independently thereof R¹ and R² are identical or different and represent hydrogen, methyl, ethyl, propyl, i-propyl, n-butyl, i-butyl, tert-butyl, n-pentyl, i-pentyl, n-hexyl, i-hexyl, n-heptyl, i-heptyl, n-octyl, i-octyl, n-nonyl, i-nonyl, n-decyl or i-decyl, preferably hydrogen or methyl.

4. Substituted cinnamic acids and cinnamic acid esters according to Claim 1, **characterized in that** they are methyl 4-chloro-2-(3-methoxy-3-oxo-1-propenyl)benzoate, 4-chloro-2-(3-methoxy-3-oxo-1-propenyl)-benzoic acid, 4-fluoro-2-(3-methoxy-3-oxo-1-propenyl)benzoic acid, methyl 4-chloro-2-(3-hydroxy-3-oxo-1-propenyl)benzoate or 4-chloro-2-(3-hydroxy-3-oxo-1-propenyl)benzoic acid.

5. Process for preparing the substituted cinnamic acids and cinnamic acid esters of the formula (I) by reacting diazonium salts of the formula (II) with acrylic acid derivatives of the formula (III) in the presence of a palladium-containing catalyst, where X, R¹ and R² are as defined in formula (I) and A^{⊖} represents halide, preferably chloride or bromide, sulphate, hydrogen sulphate, nitrate, phosphate, acetate or tetrafluoroborate, **characterized in that** the reaction is carried out in the absence of bases

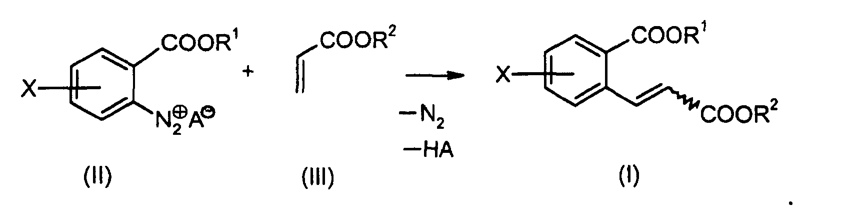

6. Process according to Claim 5, **characterized in that** the palladium-containing catalyst used is a palladium(II)salt, preferably PdCl₂, PdBr₂, Pd(NO₃)₂, H₂PdCl₄, Pd(CH₃COO)₂, [PdCl₄]Na₂, [PdCl₄]Li₂, [PdCl₄]K₂, or palladium(II) acetylacetonate.

7. Process according to Claim 5 or 6, **characterized in that** from 0.001 to 10 mol% of the palladium-containing catalyst, based on the diazonium salt of the formula (II), are employed and, independently thereof, the process is carried out at a temperature of from -20 to 100°C, preferably from 20 to 80°C and in particular from 40 to 65°C.

8. Use of the substituted cinnamic acids and cinnamic acid esters according to one or more of Claims 1 to 4 for preparing substituted indanonecarboxylic acid esters of the formula (VII) 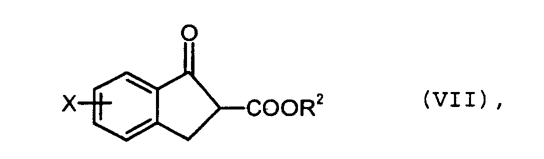 where X and R² in the formula (VII) have the meanings mentioned for the formula (I).

9. Use according to Claim 8, **characterized in that** the substituted cinnamic acids and cinnamic acid esters of the formula (I) are, in a first step, hydrogenated with hydrogen in the presence of a hydrogenation catalyst, with formation of substituted arylpropionic acids of the formula (VIII), 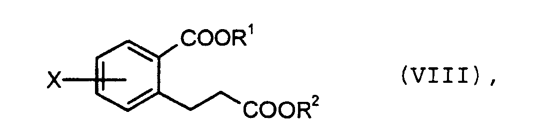 which are then, in a second step, cyclized in the presence of a base, with formation of the substituted indanonecarboxylic acid esters of the formula (VII), where X, R¹ and R² in the formulae (VII) and (VIII) each have the meanings mentioned for the formula (I).

10. Use according to Claim 9, **characterized in that** the hydrogenation is carried out under a pressure of from 1 to 100 bar and the cyclization of the substituted arylpropionic acids of the formula (VIII) is carried out in the presence of an alkali metal hydride or an alkali metal alkoxide as base.

## Revendications

1. Acides cinnamiques et esters cinnamiques substitués de formule (I) 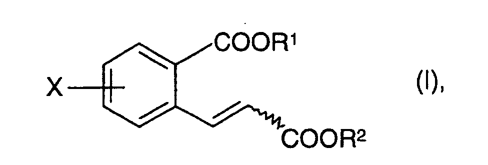 dans laquelle X représente F, Cl ou I et R¹ et R², ayant des significations identiques ou différentes, représentent chacun l'hydrogène, un groupe alkyle en C₁ à C₁₀ ou un groupe benzyle.

2. Acides cinnamiques et esters cinnamiques substitués selon la revendication 1, **caractérisés en ce que** le groupe alkyle en C₁ à C₁₀ représenté par R¹ ou R² est substitué par des halogènes, des groupes hydroxy ou aryle en C₆ à C₁₂ et, indépendamment, le groupe benzyle représenté par R¹ ou R² est substitué par des halogènes, des groupes hydroxy, alkyle en C₁ à C₁₀ ou aryle en C₆ à C₁₂.

3. Acides cinnamiques et esters cinnamiques substitués selon la revendication 2, **caractérisés en ce que** X représente le chlore et, indépendamment, R¹ et R², ayant des significations identiques ou différentes, représentent chacun l'hydrogène, un groupe méthyle, éthyle, propyle, isopropyle, n-butyle, isobutyle, tert-butyle, n-pentyle, isopentyle, n-hexyle, isohexyle, n-heptyle, isoheptyle, n-octyle, isooctyle, n-nonyle, isononyle, n-décyle ou isodécyle, de préférence l'hydrogène ou un groupe méthyle.

4. Acides cinnamiques et esters cinnamiques substitués selon la revendication 1, **caractérisés en ce qu'**ils consistent en le 4-chloro-2-(3-méthoxy-3-oxo-1-propényl)benzoate de méthyle, l'acide 4-chloro-2-(3-méthoxy-3-oxo-1-propényl)benzoïque, l'acide 4-fluoro-2-(3-méthoxy-3-oxo-1-propényl)benzoïque, le 4-chloro-2-(3-hydroxy-3-oxo-1-propényl)benzoate de méthyle ou l'acide 4-chloro-2-(3-hydroxy-3-oxo-1-propényl)benzoïque.

5. Procédé pour la préparation des acides cinnamiques et esters cinnamiques substitués de formule (I) par réaction de sels de diazonium de formule (II) avec des dérivés de l'acide acrylique de formule (III), en présence d'un catalyseur contenant du palladium, X, R¹ et R² ayant les significations indiquées en référence à la formule (I) et A^{⊖} représentant un 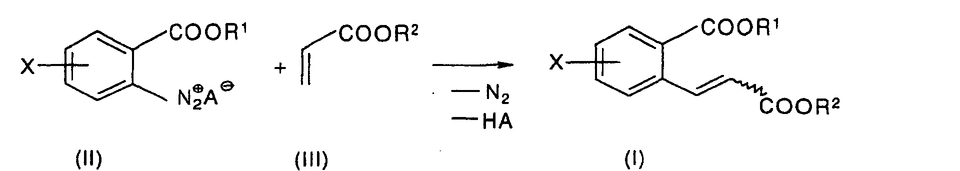 halogénure, de préférence un chlorure ou un bromure, un sulfate, un bisulfate, un nitrate, un phosphate, un acétate ou un tétrafluoroborate, **caractérisé en ce que** la réaction est réalisée en l'absence de bases

6. Procédé selon la revendication 5, **caractérisé en ce que** l'on utilise en tant que catalyseurs contenant du palladium, des sels de palladium-(II), de préférence PdCl₂, PdBr₂, Pd(NO₃)₂, H₂PdCl₄, Pd(CH₃COO)₂, [PdCl₄]Na₂, [PdCl₄]Li₂, [PdCl₄]K₂ ou l'acétylacétonate de palladium-(II).

7. Procédé selon la revendication 5 ou 6, **caractérisé en ce que** l'on utilise de 0,001 à 10 mol % du catalyseur contenant du palladium par rapport au sel de diazonium de formule (II) et que, indépendamment, on opère à une température comprise entre -20 et 100°C, de préférence entre 20 et 80°C et plus spécialement entre 40 et 65°C.

8. Utilisation des acides cinnamiques et esters cinnamiques substitués selon une ou plusieurs des revendications 1 à 4 pour la préparation des esters indanone-carboxyliques substitués de formule (VII) 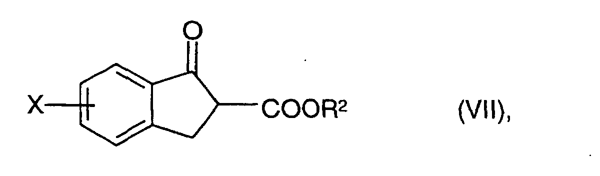 dans laquelle X et R² de la formule (VII) ont les significations indiquées en référence à la formule (I).

9. Utilisation selon la revendication 8, **caractérisée en ce que**, dans un premier stade, les acides cinnamiques et esters cinnamiques substitués de formule (I) sont hydrogénés en présence d'un catalyseur d'hydrogénation, ce qui donne les acides arylpropioniques substitués de formule (VIII), 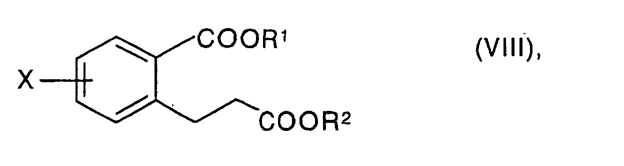 qu'on cyclise dans un deuxième stade, en présence d'une base, en les esters indanone-carboxyliques substitués de formule (VII), les symboles X, R¹ et R² ayant dans les formules (VII) et (VIII) les significations indiquées en référence à la formule (I).

10. Utilisation selon la revendication 9, caractérisée en ce l'hydrogénation est réalisée sous une pression de 1 à 100 bar et la cyclisation des acides arylpropioniques substitués de formule (VIII) est réalisée en présence d'un hydrure de métal alcalin ou d'un alcoolate de métal alcalin constituant la base.
